# EUROPEAN PATENT SPECIFICATION

(11) **EP 1 891 880 B1**
(45) Date of publication and mention of the grant of the patent: **01.05.2013**
(21) Application number: 06729285.4
(22) Date of filing: 16.03.2006
(51) Int. Cl.: A61B 1/00, A61B 17/00, A61B 17/28, G02B 23/24

(54) **ENDOSCOPE**
ENDOSKOP
ENDOSCOPE

(30) Priority: 26.04.2005 JP 2005127580
(43) Date of publication of application: 27.02.2008
(73) Proprietor: OLYMPUS MEDICAL SYSTEMS CORP., Tokyo 151-0072 (JP)
(72) Inventor: OKADA, Tsutomu, 1900012 (JP); YAMAMOTO, Tetsuya, 3570044 (JP); KURA, Yasuhito, 1920363 (JP)
(74) Representative: von Hellfeld, Axel
(86) International application number: PCT/JP2006/305292
(87) International publication number: WO 2006/117937

(56) References cited:
- JP-A- 60 242 417
- JP-A- 2003 220 022
- JP-A- 2004 194 827
- US-B1- 6 352 503

## Description

### TECHNICAL FIELD

The present invention relates to an endoscope apparatus which is used for medical use or industrial use so as to perform various treatments on a treatment target portion.

Priority is claimed on Japanese Patent Application No. 2005-127580, filed April 26, 2005, the content of which is incorporated herein by reference.

### BACKGROUND ART

Recently, various endoscope apparatuses have been utilized in various fields such as medical fields, industrial fields and the like. In general, each of those endoscope apparatuses includes an endoscope insertion portion extending in an elongated shape and an endoscope main body supporting the endoscope insertion portion.

Among the endoscope apparatuses, an endoscope apparatus including a ring member is proposed, the ring being capable of projecting or retreating from a distal end surface of the endoscope insertion portion extending in a cylinder shape (for example, refer to Patent Document 1: Japanese Unexamined Patent Application, First Publication No. 11-299726).

Further, instead of the ring member, an endoscope apparatus including a hood formed in a cylindrical shape is proposed, instead of the ring member (for example, refer to Patent Document 2: Japanese Unexamined Patent Application, First Publication No. 2-124438).

In these endoscope apparatuses, a distance between a distal end surface of the endoscope insertion portion and a treatment target portion can be secured in such a manner that the treatment target portion can be prevented from coming contact with the distal end surface or the like.
Patent Document 1: Japanese Unexamined Patent Application, First Publication No. H11-299726
Patent Document 2: Japanese Unexamined Patent Application, First Publication No. H2-124438

Further, the document JP2003220022 discloses an endoscope that comprises a pressing member having a T-shape disposed at the distal end surface of the endoscope insertion portion so as to be retreated from the projection position, the pressing member is projected from a projection opening formed in the distal end surface of the endoscope insertion portion.

### DISCLOSURE OF THE INVENTION

### PROBLEM TO BE SOLVED BY THE INVENTION

In the endoscope apparatuses disclosed in Japanese Unexamined Patent Application, First Publication Nos. 11-299726 (Patent Document 1) and 2-124438 (Patent Document 2), however, when a tissue or the like which gets in the way (causes an obstruction) during treatment is formed in the vicinity of the treatment target portion, it is difficult to perform a proper treatment because of the interference of the tissue or the like, even through the distance can be secured.

An advantage of the invention is that it provides an endoscope apparatus which can easily secure a treatment region even through an obstructing tissue is present in the vicinity of a treatment target portion. Therefore, it is possible to easily and rapidly perform a treatment depending on various conditions.

### MEANS FOR SOLVING THE PROBLEM

The present invention is defined in claim 1.

In the endoscope apparatus according to this aspect, when various treatments are not performed, like when the endoscope insertion portion is fed to the treatment target portion, the pressing member is disposed in the retreat position. On the other hand, when various treatments are performed, the pressing member is disposed in the projection position where it is projected from the distal end surface, in a state where a position in the lower side from the observation window and in the lower side from the channel opening on the distal end surface is set to a base end portion, or a position in the upper side of the observation window and the upper side from the channel opening on the distal end surface is set to a base end portion. Therefore, when the pressing member is disposed in the projection position, it is projected from the lower or upper side from the observation window or the channel opening.

Accordingly, even when a portion adjacent to the treatment target portion gets in the way, the portion can be pressed by the pressing members such that a treatment region can be easily secured.

'Pressing' of the pressing member is referring to as pressing or supporting a position adjacent to the treatment target portion.

According to a second aspect of the invention, in the endoscope apparatus according to the first aspect, the retreat position is a withdrawal position where the pressing member is withdrawn into the endoscope insertion portion, and the pressing member is provided to reciprocate between the projection position and the withdrawal position, and the pressing member is provided to reciprocate between the projection position and the withdrawal position.

In the endoscope apparatus according to this aspect, the pressing member reciprocates between the projection position and the withdrawal position.

Accordingly, the pressing member can be easily and reliably projected and withdrawn from the distal end surface. Further, as the pressing member is advanced and retreated from the distal end surface, the pressing member can be easily disposed to a portion adjacent to the treatment target portion, without moving the endoscope insertion portion.

According to the present invention, the channel opening is provided in the lower side of the observation window, and the pressing member is projected from the position in the lower side of the channel opening.

In the endoscope apparatus according to this aspect, when the pressing member is disposed in the projection position, the pressing member is projected from the lower side of the channel opening. At this time, the observation window, the channel opening, and the pressing member are sequentially disposed from the upper side of the distal end surface.

Accordingly, when the portion adjacent to the treatment target portion is pressed downwardly by the pressing member, an observed region from the observation window and a treatment region of the treatment instrument from the channel opening can be disposed in the same side with respect to the pressing member. Therefore, it is possible to easily perform the treatment while the treatment region is observed.

According to the present invention, the pressing member is constructed in such a manner that the rigidity thereof in the vertical direction on the distal end surface is higher than that in a direction crossing the vertical direction on the distal end surface.

In the endoscope apparatus according to this aspect, since the pressing member is constructed in such a manner that its rigidity in the vertical direction increases, the pressing force for pressing a portion adjacent to the treatment target portion in the vertical direction or the support force for supporting a portion adjacent to the treatment target portion in the vertical direction can be increased. Therefore, it is possible to easily press the portion adjacent to the treatment target portion.

### EFFECTS OF THE INVENTION

According to the present invention, even when an obstructing tissue is present, the obstructing tissue can be easily pressed by the pressing members. Therefore, it is possible to easily secure the treatment region and to easily and rapidly perform a treatment depending on various conditions.

### BRIEF DESCRIPTION OF THE DRAWINGS

FIG. 1 is an overall view of an endoscope apparatus according to an embodiment of the present invention.
FIG 2 is a front view of a distal end surface of an insertion portion.
FIG 3 is a diagram for explaining an observed image during treatment.
FIG 4 is a diagram showing the appearance of the distal end of the insertion portion, showing a state where pressing rods are disposed in a withdrawal position.
FIG 5 is a diagram showing the appearance of the distal end of the insertion portion, showing a state where pressing rods are disposed in a projection position.
FIG 6 is a cross-sectional view of the pressing rods.
FIG 7 is a diagram showing a state where an excision treatment is performed using the endoscope apparatus and an endoscope treatment instrument, and the insertion portion is disposed in the vicinity of the front side of a fold.
FIG 8 is a diagram showing a state where the excision treatment is performed using the endoscope apparatus and the endoscope treatment instrument, and the fold is pressed by the pressing rods.
FIG 9 is a diagram showing a state where the excision treatment is performed using the endoscope apparatus and the endoscope treatment instrument, and a polyp is excised in a state where the fold is pressed by the pressing rods.
FIG 10 is a diagram showing essential parts of an endoscope according to an example not part of the present invention and is a front view of a distal end surface.
FIG 11 is a side view showing the appearance of a distal end of an insertion portion according to said example.
FIG 12 is a perspective view showing the appearance of the distal end of the insertion portion according to said example showing a state where a pressing member is disposed in a parallel position.
FIG 13 is a perspective view showing the appearance of the distal end of the insertion portion according to said example, showing a state where the pressing member is disposed in a projection position.
FIG. 14 is a diagram showing essential parts of an endoscope according to an example not part of the present invention and is a front view of a distal end surface.
Fig .15 is a perspective view showing the appearance of the distal end of an insertion portion according to said example, showing a state where a pressing member is disposed in a parallel position.
FIG 16 is a perspective view showing the appearance of the distal end of the insertion portion according to said example, showing a state where the pressing member is disposed in a projection position.
FIG 17 is a diagram showing a state where an excision treatment is performed using the endoscope apparatus and an endoscopic treatment instrument, and a portion adjacent to a lesion is excised.
FIG 18 is a diagram showing a state where an excision treatment is performed using the endoscope apparatus and the endoscopic treatment instrument, and the vicinity of the lesion is supported by the pressing member.
FIG. 19 is a diagram showing a state where an excision treatment is performed using the endoscope apparatus and the endoscopic treatment instrument, and the vicinity of the lesion is supported by the pressing member in a state where the portion is further excised.
FIG 20 is a diagram for explaining an observed image during treatment according to said example.
FIG 21 is a diagram showing essential parts of an endoscope apparatus according to an embodiment of the invention and is a perspective view of a distal end portion of an insertion portion.
FIG 22 is a diagram of a modification of the endoscopic treatment instrument.
FIG 23 is a diagram showing a state where a snare loop of the endoscopic treatment instrument of FIG 22 is pressed against an inner wall.

### DESCRIPTION OF THE REFERENCE SYMBOLS

1: ENDOSCOPE APPARATUS
2: ENDOSCOPIC TREATMENT INSTRUMENT
14: INSERTION PORTION (ENDOSCOPE INSERTION PORTION)
24: PRESSING ROD (PRESSING MEMBER)
30: TREATMENT INSTRUMENT CHANNEL
31: DISTAL END SURFACE
34: CCD (OBSERVATION UNIT)
35: OBSERVATION WINDOW
41: CHANNEL OPENING
45: POLYP (TREATMENT TARGET PORTION)
50: FOLD (A PORTION IN THE VICINITY OF A TREATMENT TARGET PORTION)
53: PRESSING FRAME (PRESSING MEMBER)
54: ROTATING SHAFT
61: LESION (TREATMENT TARGET PORTION)
64: MUCOSA (A PORTION ADJACENT TO THE TREATMENT TARGET PORTION)
A: WITHDRAWAL POSITION (RETREAT POSITION)
B: PROJECTION POSITION
H: VERTICAL DIRECTION (VERTICAL DIRECTION OF THE DISTAL END SURFACE)
H_{U}: UPPER SIDE ON THE DISTAL END SURFACE
H_{D}: LOWER SIDE ON THE DISTAL END SURFACE

### BEST MODE FOR CARRYING OUT THE INVENTION

Hereinafter, an endoscope apparatus according to a first embodiment of the invention will be described with reference to the drawings.

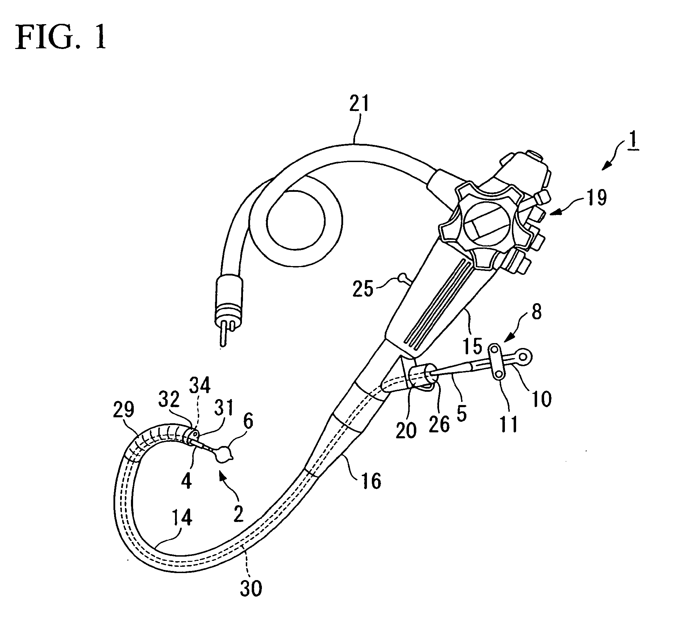

As shown in FIG 1, the endoscope apparatus according to this embodiment can perform various treatments using an endoscopic treatment instrument 2.

First, the endoscopic treatment instrument 2 will be described.

The endoscopic treatment instrument 2 includes a flexible sheath 5, which extends in a cylindrical shape, and an operation wire 4 inserted into the flexible sheath 5. The flexible sheath 5 has an operator 8 provided in a base end portion of the flexible sheath 5. The operator 8 includes an operation shaft 10 extending in an axial direction of the flexible sheath 5 and a slider 11 supported by the operating shaft 10 such that the slider 11 can advance and retreat. Meanwhile, a distal end portion of the flexible sheath 5 is set to an opened end. Further, the operation wire 4 has a snare loop 6 provided at the distal end portion thereof and the slider 11 attached to the base end portion thereof. The snare loop 6 composed of an elastic wire is formed in a loop shape. In such a structure, when the slider 11 is advanced and retreated, the snare loop 6 is projected and withdrawn from the distal end of the flexible sheath 5 through the operation wire 4.

Next, the endoscope apparatus 1 according to the invention will be described.

The endoscope apparatus 1 includes a long insertion portion (endoscope insertion portion) 14, which is inserted into the body, and a main body operator 55 which is connected to the insertion portion 14 so as to support the insertion portion 14. In a connecting portion of the insertion portion 14 and the main body operator 15, a break prevention portion 16 is provided.

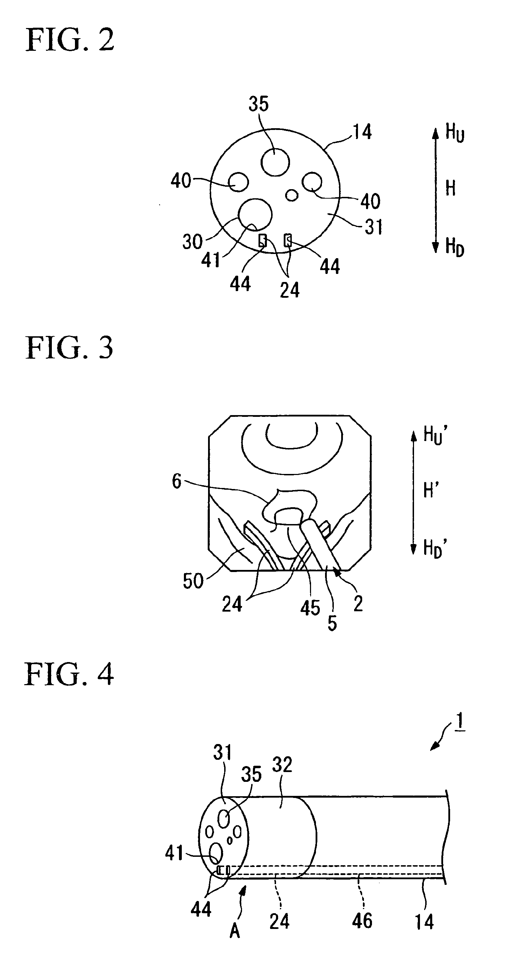

The insertion portion 14 includes a curved portion 29 which can be curved. As the curved portion 29 is operated so as to be curved, the distal end of the insertion portion 14 can be directed to a desirable direction. Inside the insertion portion 14, a treatment instrument channel 30 is provided, which serves as an insertion path of the endoscopic treatment instrument 2. Inside a distal end portion 32 of the insertion portion 14, a charge coupled device (CCD) 34 is provided, which serves as an observation unit. Further, as shown in FIG 2, the insertion portion 14 has an observation window 35 formed on a distal end surface 31 thereof such that the observation window 35 faces the CCD 34. Further, between the CCD 34 and the observation window 35, an object lens (not shown) is provided.

Further, on the distal end surface 31, a lighting portion 40 for irradiating illumination light and a channel opening 41 continuing to the treatment instrument channel 30 are formed.

As shown in FIG. 1, the above-described main body operator 15 includes an operator 19 for performing various operations and a universal cord 21 for connecting the operator 19 to an apparatus main body (not shown) having a monitor or the like. Further, the main body operator 15 includes an operation lever 25 for operating a pair of pressing rods 24 (shown in FIG 2) and a forceps plug 20 for inserting the endoscopic treatment instrument 2. The forceps plug 20 has a forceps plug opening 26 for inserting the endoscopic treatment instrument 2. The forceps plug opening 26 communicates with a channel opening 41 (shown in FIG 2) through the treatment instrument channel 30. That is, the channel opening 41 is formed in the distal end side of the treatment instrument channel 30, and the forceps plug opening 26 is formed in the base end side (hand side).

On the distal end surface 31 according to this embodiment, a pair of projection and withdrawal openings 44, through which the pressing rods (pressing members) 24 are projected and withdrawn, are formed in the vicinity of a peripheral portion in a lower side H_{D} of the distal end surface 31. The lower side H_{D} on the distal end surface 31, shown in FIG 2, indicates a direction corresponding to a lower side H_{D}' in a vertical direction H' of an observed image shown in FIG 3, the observed image being obtained when treatment is performed using the endoscopic treatment instrument 2. An upper side H_{U} on the distal end surface 31 indicates a direction corresponding to an upper side H_{U}' in the vertical direction H' of the observed image. Further, when treatment is performed using the endoscopic treatment instrument 2, the rotational position 14 of the insertion portion around its axis line is adjusted in such a manner that a treatment target portion such as a polyp 45 or the like is disposed in the lower side H_{D}' of the observed image.

Further, on the distal end surface 31, the above-described observation window 35 is provided in the vicinity of the peripheral portion in the upper side H_{U} thereof. The channel opening 41 is formed in the lower side H_{D} of the observation window 35, and the projection and withdrawal opening 44 is formed in the lower side H_{D} of the channel opening 41.

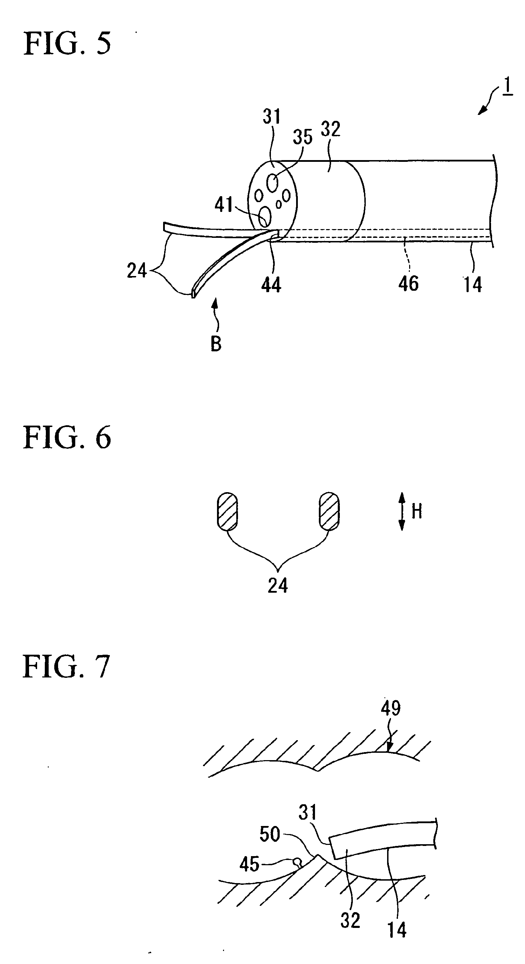

As shown in FIG. 4, the pair of plate-shaped pressing rods 24 which are formed of an elastic member are provided on the distal end portion 32 of the insertion portion 14. Further, as shown in FIG. 5, the pair of pressing rods 24 tend to be bent from the base end toward the distal end in such a manner that the space between the pressing rods 24 is gradually widened in a direction where they are separated from each other. As shown in FIG 6, the lateral cross-sections of the pressing rods 24 are formed in a vertically long shape extending in the vertical direction H. The rigidity thereof in the vertical direction is set to be higher than that in the side-to-side direction. The side-to-side direction indicates a direction crossing the vertical direction H at right angles.

As shown in FIG. 4, the base end portions of the pressing rods 24 are fixed to a distal end of a rigid wire 46. A rear end of the rigid wire 46 is attached to the operation lever 25 shown in FIG. 1. In such a structure, when the operation lever 25 is operated, the pressing rods 25 are projected and withdrawn from the distal end surface 31 through the rigid wire 46. That is, when the operation lever 25 is pulled by a predetermined amount, the pressing rods 24 are disposed in a withdrawal position (retreat position) A where they are withdrawn into the distal end portion 32. On the other hand, when the operation lever 25 is pushed by a predetermined amount, the pressing rods 24 are disposed in a projection position B where they are projected from the distal end surface 31 toward the outside of the insertion portion 14 in an axial direction thereof. As such, the pressing rods 24 are constructed to reciprocate between the projection position B and the withdrawal position A. That is, when the pressing rods 24 are disposed in the withdrawal position A, they are retreated from the projection position B. On the other hand, when the pressing rods 24 are disposed in the projection position B, they are projected from the distal end surface 31, in a state where a position in the lower side H_{D} from the observation window 35 and in the lower side H_{D} from the channel opening 41, that is, the projection and withdrawal opening 44 is set to the base end portion.

Next, a method of using the endoscope apparatus I according to this embodiment of the invention will be described. In this embodiment, as shown in FIGS. 7 to 9, a treatment will be exemplified, where a polyp 45 formed on the inner wall of a large intestine 49 is excised.

In this embodiment, it is assumed that a fold (a portion in the vicinity of a treatment target portion) 50 is formed on the inner wall of the large intestine 49, the insertion portion 14 is inserted from the near side of the fold 50, and the polyp 45 is formed in the inner side of the base end portion of the fold 50.

First, the operation lever 25 is operated to dispose the pressing rods 24 in the withdrawal position A. Then, the insertion portion 14 is inserted into the large intestine 49. While a captured image obtained by the CCD 34 is observed through the observation window 35, the insertion portion 14 is fed until the distal end portion 32 of the insertion portion 32 is disposed in the vicinity of the near side of the fold 50, as shown in FIG. 7. Then, the insertion portion 14 is rotated around its axis line in such a manner that the fold 50 is disposed in the lower side H_{D}' of the observed image. The vertical direction H', when the fold 50 is disposed in the lower side H_{D}' of the observed image, corresponds to the vertical direction H of the distal end surface 31, as described above.

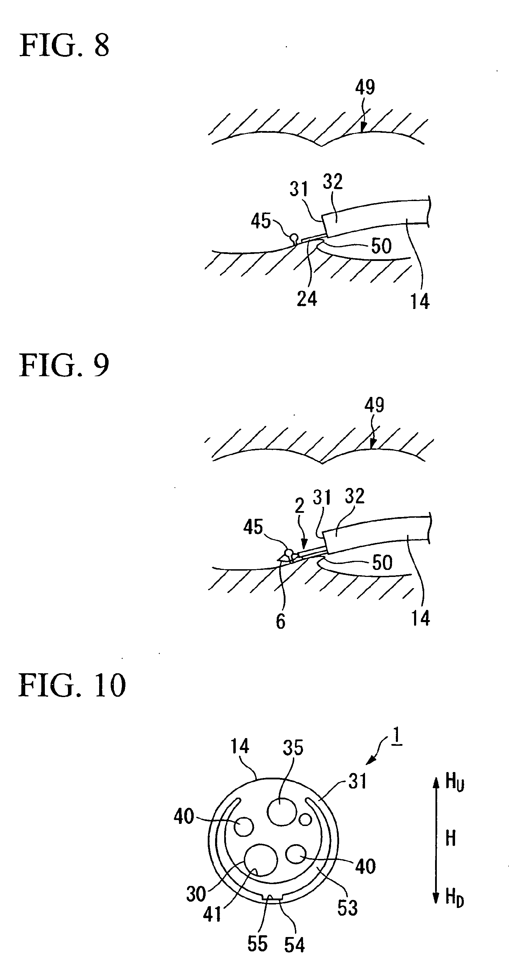

At this time, since the fold 50 stands in front of the distal end surface 31, the fold 50 gets in the way of (obstructs) the observed image such that the polyp 45 in the inner side of the fold 50 is not shown. Further, since a treatment region from which the polyp 45 is excised is not secured, the treatment cannot be performed. Therefore, in order to remove the fold 50 from the observed image and to secure the treatment region, the fold 50 needs to be pressed. Thus, the operation lever 25 is operated to dispose the pressing rods 24 in the projection position B. As described above, the pressing rods 24 tend to be bent from the base end toward the distal end such that the space therebetween is gradually widened. Therefore, as the projection dimensions of the pressing rods 24 from the distal end surface 31 increase, the space therebetween is gradually widened, as shown in FIG 5. In this state where the pressing rods 24 are projected from the distal end surface 31 such that the space therebetween is widened, the upper end of the fold 50 is pressed downward by the pressing rods 24, as shown in FIG 8. Then, the fold 50 is pressed against the inner wall of the large intestine 49. As such, the fold 50 is pressed by the pressing rods 24.

At this time, since the fold 50 is pressed, the fold 50 is removed from the observed image. Then, the front side of the distal end surface 31 is opened in such a manner that a treatment region and an observed region are secured. Therefore, as shown in FIG 3, the polyp 45 is shown on the observed image. In this state, the flexible sheath 5 of the endoscopic treatment instrument 2 is inserted into the treatment instrument channel 30, and the distal end of the flexible sheath 5 is projected from the distal end surface 31. Further, the slider 11 is advanced so that the snare loop 6 is projected from the distal end of the flexible sheath 5. Then, as shown in FIG. 9, the polyp 45 is caught into the snare loop 6. After that, when the slider 11 is retreated, the snare loop 6 is folded so as to be withdrawn from the distal end of the flexible sheath 5. Then, the polyp 45 within the snare loop 6 is constricted. In this state, when a high-frequency current is applied, the polyp 45 is excised. Then, as the excised polyp 45 is collected, the series of excision treatments of the polyp 45 are terminated.

According to the above-described endoscope apparatus 1 of this embodiment, the fold 50 can be easily pressed by the pressing rods 24, and the treatment region can be easily secured. Therefore, the treatment can be easily and rapidly performed depending on various conditions.

Further, although the pressing rods 24 are disposed in the projection position B, the pressing rods 24 are projected from the distal end surface 31, in a state where the position in the lower side H_{D} from the observation window 35 and in the lower side H_{D} from the channel opening 41 is set to the base end portion. Therefore, the treatment region and the observed region are prevented from being blocked by the pressing rods 24. Further, the pressing rods 24 can be prevented from interfering with the endoscopic treatment instrument 2. Therefore, when the treatment is performed, the treatment region can be reliably secured, which makes it possible to easily perform the treatment.

In addition, when the pressing rods 24 are disposed in the projection position B, the space between the pressing rods 24 is widened. Therefore, the fold 50 can be reliably pressed across a wide area. Further, as the projection dimensions of the pressing rods 24 projecting from the distal end 31 are adjusted, the widening degree of the pressing rods 24 can be adjusted so that the pressing rods can correspond to various treatment target portions.

As the pressing rods 24 are advanced and retreated from the distal end surface 31, it is possible to easily dispose the pressing rods 24 to the fold 50, without moving the insertion portion 14.

The channel opening 41 is formed in the lower side H_{D} of the observation window 35, and the pressing rods 24 are projected from the lower side H_{D} of the channel opening 41. Therefore, since the pressing rods 24 are not disposed between the observation window 35 and the channel opening 41, the pressing rods 24 can be prevented from interfering with the treatment performed by the endoscopic treatment instrument 2. That is, the observed region from the observation window 35 and the treatment region of the endoscopic treatment instrument 2 projected from the channel opening 41 can be disposed in the same side with respect to the pressing rods 24. Therefore, while the treatment region is observed, the treatment can be easily performed.

The pressing rods 24 are constructed in such a manner that the rigidity thereof in the vertical direction H increases. Therefore, the pressing force of the pressing rods 24, when pressing the fold 50 in the vertical direction H, can be increased. Accordingly, the fold 50 or the like can be easily pressed.

In this embodiment, the pressing rods 24 tend to be bent in such a manner that the space therebetween is widened. Without being limited thereto, the space between the pressing rods 24 may be widened by a biasing member such as a spring.

Hereinafter, an example which is not part of the present invention will be described.

FIGS. 10 to 13 illustrate said example.

In FIGS. 10 to 13, like reference numerals are attached to the same components as those of FIGS. 1 to 9, and the descriptions thereof will be omitted.

The basic construction of this example is almost the same as that of the first embodiment, and thus only different aspects will be described.

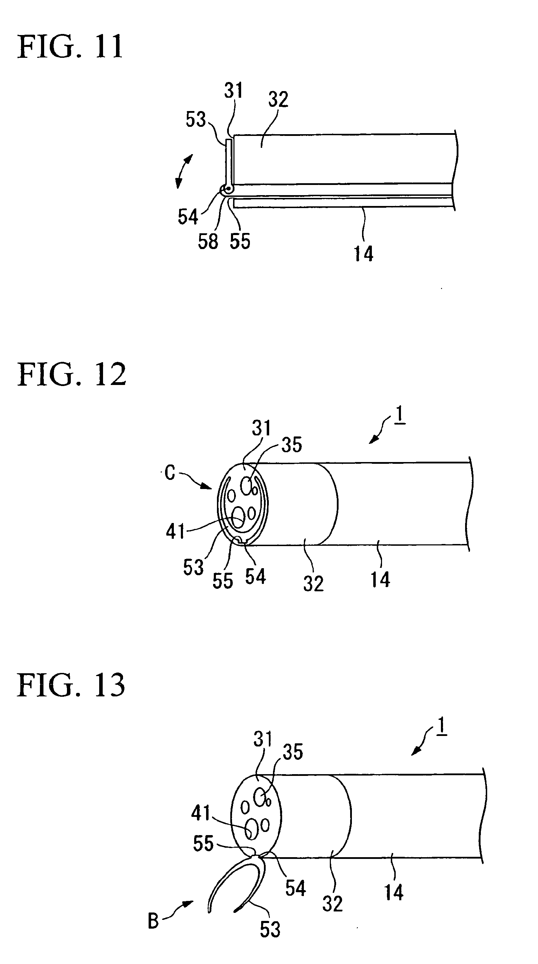

As shown in FIG 10, an endoscope apparatus 1 according to this example includes a pressing frame (pressing member) 53 formed in a substantially semi-circular frame shape. In the central portion of the pressing frame 53 in a circumferential direction thereof, a rotating shaft 54 is provided. The rotating shaft 54 is formed to extend in a line along the distal end surface of the insertion portion 14 and is disposed in a projection opening 55 formed in the vicinity of a peripheral portion in the lower side H_{D} of the distal end surface 31. Accordingly, the pressing frame 53 is attached so as to rotate about the rotating shaft 54. Further, the pressing frame 53 is biased in a direction approaching the distal end surface by a biasing member (not shown). Therefore, the pressing frame 53 is disposed adjacent to the distal end surface 31 in a normal state. More specifically, the pressing frame 53 is disposed in a parallel position C (shown in FIG 12) at which the pressing frame 53 extends in a plane shape along the distal end surface 31.

As shown in FIG 11, the pressing frame 53 is fixed to the distal end of a rotation operation wire 58 in the front surface side thereof. The base end portion of the rotation operation wire 58 extending into the insertion portion 14 through the lower side H_{D} of the pressing frame 53 is attached to the same operation lever 25 as that of the embodiment.

On the distal end surface 31, the observation window 35, the channel opening window 41, and the projection opening 55 are sequentially formed from the upper side H_{U}. Such a structure is the same as that of the embodiment.

In such a structure, as the operation lever 25 is operated, the pressing frame 53 is rotated about the rotating shaft 54 so as to be opened and closed with respect to the distal end surface 31. That is, when the operation lever 25 is pulled by a predetermined amount, the pressing frame 53 is pulled in a direction away from the distal end surface 31 through the rotation operation wire 58. Thus, the pressing frame is rotated about the rotating shaft 54 against the biasing force of the biasing member. Then, when the pressing frame 53 is disposed so as to be projected from the distal end surface 31 toward the outside of the axial direction of the insertion portion 14, the position of the pressing frame 53 in this state is referred to as a projection position B shown in FIG 13. Meanwhile, when the operation lever 25 is released, the pressing frame 53 is disposed in the parallel position (retreat position) C by the biasing force of the biasing member. As such, the pressing frame 53 is constructed so as to rotate about the rotating shaft 54, between the projection position B and the parallel position C. Further, similar to the first embodiment, when the pressing frame 53 is disposed in the projection position B, it projects from the distal end surface 31 in a position of the lower side H_{D} from the observation window 35 and of the lower side H_{D} from the channel opening 41, that is, in a state where the projection opening 55 is set to the base end portion.

In such a structure, the insertion portion 14 is disposed in the vicinity of the near side of the fold 50, similar to the embodiment. Then, as the operation lever 25 is pulled by a predetermined amount, the pressing frame 53 is disposed in the projection position B. Accordingly, the fold 50 is pressed by the pressing frame 52, and the polyp 45 is shown on an observed image. After that, the polyp 45 is excised by the same manner as the embodiment.

In this embodiment, when the treatment is performed, a treatment region and an observed region can be reliably secured so as to easily perform the treatment. Further, as the rotational angle of the pressing frame 53 is changed, the position at which the fold 50 is pressed can be adjusted. Therefore, it is possible to adjust the width of the treatment region, depending on various conditions.

Hereinafter, an example which is not part of the present invention will be described.

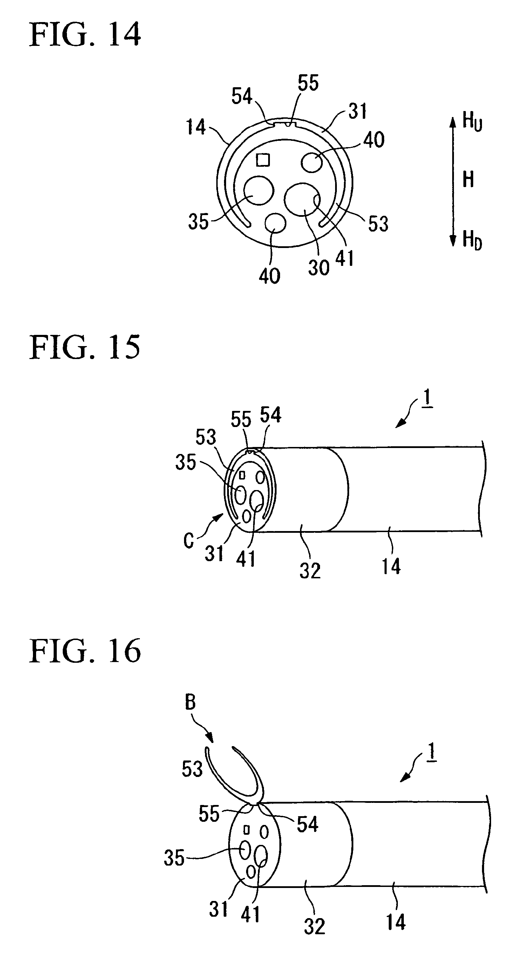

FIGS. 14 to 20 illustrate a third embodiment of the invention.

As shown in FIG 14, in an endoscope apparatus 1 according to this example, the projection opening 55 is formed in the vicinity of the peripheral portion of the distal end surface 31 in the upper side H_{U}, the observation window 35 is provided in the lower side H_{D} of the projection opening 55, and the channel opening 41 is formed in the lower side H_{D} of the observation window 35.

As the operation lever 25 is operated, the pressing frame 53 is opened and closed with respect to the distal end surface 31, while rotating about the rotating shaft 54. That is, as shown in FIGS. 15 and 16, the pressing frame 53 is constructed to rotate about the rotating shaft 54, between the projection position B and the parallel position C. Further, when the pressing frame 53 is disposed in the projection position B, it is projected from the distal end surface 31 in a position in the upper side H_{U} from the observation window 35 and in the upper side H_{U} from the channel opening 41, that is, in a state where the projection opening 55 is set to the base end portion.

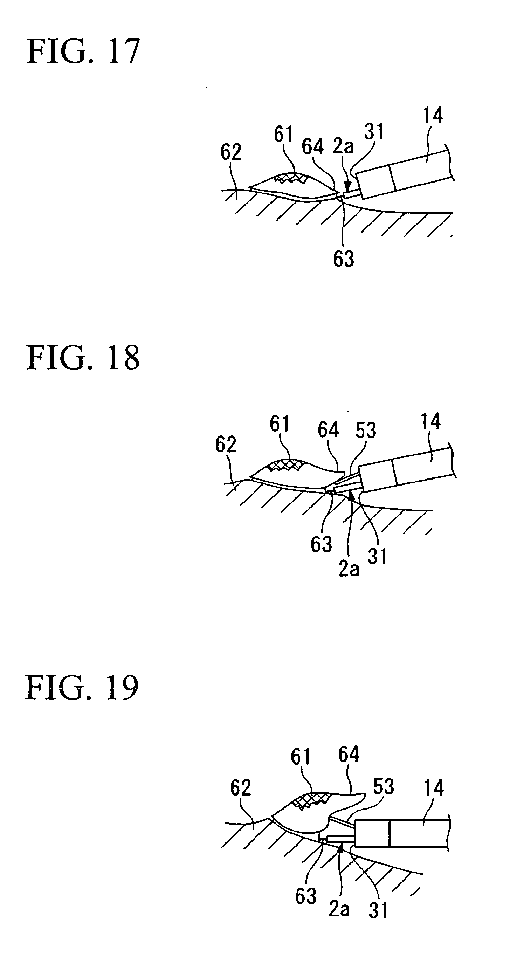

A method of using the endoscope apparatus 1 constructed in such a manner will be described. In this example, as shown in FIGS. 17 to 20, a treatment will be exemplified, in which a lesion (treatment target portion) 61 formed on the inner wall of a stomach 62 is excised.

In this example, an endoscopic treatment instrument 2a having an incision electrode 63 formed at the distal end of thereof is used. The endoscopic treatment instrument 2a is a so-called high-frequency knife. As a high-frequency voltage is applied to the incision electrode 63, a living tissue can be excised.

Similar to the embodiment, the insertion portion 14 is fed to the vicinity of the front side of the lesion 61. Then, while a captured image is observed, a tissue adjacent to the lesion 61 is excised by the incision electrode 63 little by little, as shown in FIG. 17. Then, a mucosa (a portion adjacent to the treatment target portion) 64 separated from the inner wall by the incision electrode 63 is placed on the endoscopic treatment instrument 2a due to its weight. Therefore, as shown in FIG. 18, when the pressing frame 53 is projected from the distal end surface 31 before the separated mucosa 64 is placed on the endoscopic treatment instrument 2a, the separated mucosa 64 is placed on the pressing frame 53. At this time, the separated mucosa 64 is supported by the pressing frame 53. More specifically, the separated mucosa 64, which droops downward, is pressed by the pressing frame 53. Therefore, the treatment region and the observed region can be secured.

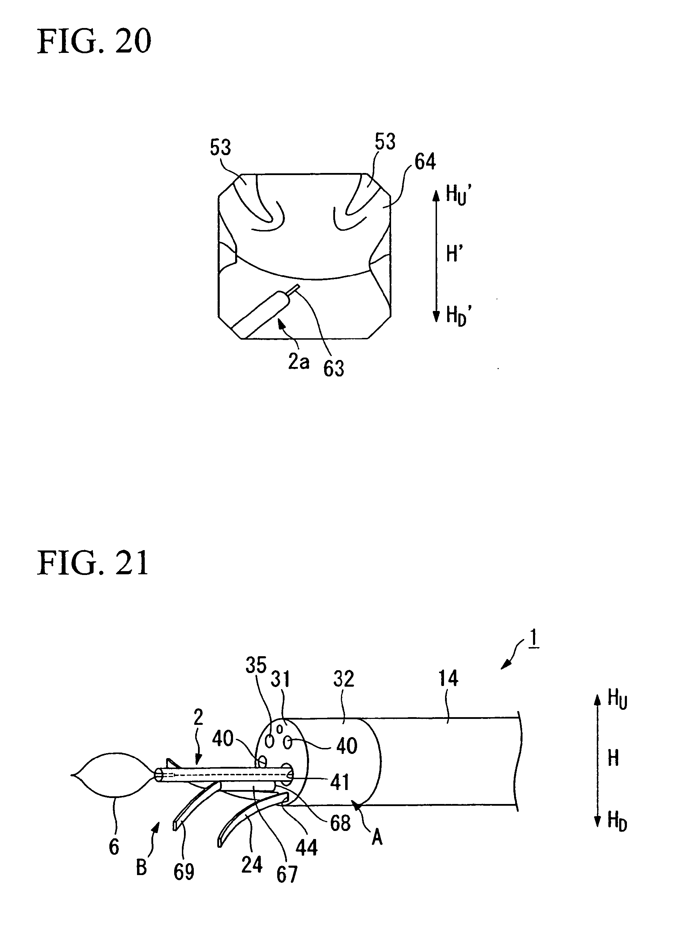

As the excision is promoted, the area of the separated mucosa 64 placed on the pressing frame 53 increases. Therefore, the treatment region and the observed region are gradually narrowed. At this time, the separated mucosa 64 needs to be supported more upwardly, in order to secure a wide treatment region and observed region. Therefore, as shown in FIG. 19, the operation lever 25 is further pulled in such a manner that the distal end of the pressing frame 53 is positioned more upwardly. That is, the open angle of the pressing frame 53 is adjusted in such a manner that the angle between the pressing frame 53 and the distal end surface 31 increases. Then, as shown in FIG. 20, the wider treatment region and observed region are secured.

As described above, when the treatment is performed, the treatment region and the observed region can be reliably secured. Therefore, it is possible to easily perform the treatment.

Since the pressing frame 53, the observation window 35, and the channel opening 41 are sequentially disposed from the upper side of the distal end surface 31, the observed region from the observation window 35 and the treatment region of the endoscopic treatment 2 instrument projected from the channel opening 41 can be disposed in the same side with respect to the pressing frame 53. Further, since the channel opening 41 is disposed in the lower side of the observation window 35, the observed region can be sufficiently secured.

The observation opening 36 is provided in the upper side H_{U} of the channel opening 41. Without being limited thereto, however, the observation opening 36 may be provided in the lower side H_{D} of the channel opening 41. Accordingly, when a fold or the like drooping downward from the upper side is pressed against an upper wall, the space between the channel opening 41 and the observation opening 36 can be secured. Therefore, it is possible to reliably secure a visual field for observation.

When the operation lever 25 is released, the pressing frame 53 returns to the parallel position C. Without being limited thereto, however, a lock mechanism for locking the operation lever 25 may be provided. Then, although the hand is separated from the operation lever 25 during treatment, the pressing frame 53 can be locked to the projection position B, which makes it possible to easily perform the treatment.

Further, the distal end surface 31 may include a concave portion for housing the pressing frame 53. That is, when the pressing frame 53 is disposed in the parallel position C, the pressing frame 53 is set to be disposed into the concave portion. Therefore, when the pressing frame 53 is disposed in the parallel position C, it is possible to reliably retreat the pressing frame 53.

Next, a another embodiment of the invention will be described.

FIG 21 illustrates a another embodiment of the invention.

As shown in FIG 21, an endoscope apparatus 1 according to this embodiment includes a pressing forceps 67 for pressing a portion adjacent to a target treatment portion. The pressing forceps 67 has a pair of pressing portions 69 formed on the distal end thereof, the pressing portions 69 being the same as the pressing rods 24. Further, the insertion portion 14 has two channels formed therein. On the distal end surface 31, two openings continuing to the respective channels are formed. That is, one opening serves as the above-described channel opening 41, and the other opening serves as a pressing forceps opening 68.

The respective openings are formed on the distal end 31 such that the observation opening 35, the channel opening 41, the pressing forceps opening 68, and the projection and withdrawal opening 44 are sequentially formed from the upper side H_{U} to the lower side H_{D}.

In such a structure, not only are the pressing rods 24 projected and withdrawn from the distal end surface 31, but also the pressing portions 69 can be projected and withdrawn from the distal end surface 31 through the pressing forceps opening 68, as the pressing forceps 67 is advanced and retreated from the distal end surface 31. That is, the pressing portions 69 are constructed to reciprocate between the withdrawal position A, at which they are withdrawn into the distal end portion 32, and the projection position B at which they are projected from the distal end portion 32. Further, when both the pressing portions 69 and the pressing rods 24 are disposed in the projection position B, the pressing portions 69 are projected from the lower side H_{D} of the observation window 35 and the channel opening 41, and the pressing rods 24 are projected from the lower side H_{D} of the pressing portions 69.

Therefore, since even a portion which is not pressed by the pressing rods 24 can be pressed by the pressing portions 69, it is possible to reliably secure a treatment region and an observed region even in a complicated condition.

In this embodiment, both the pressing rods 24 and the pressing portions 69 are provided. Without being limited thereto, however, at least any one of them may be provided. That is, when only the pressing rods 24 are provided, the structure of this embodiment is similar to that of the first embodiment. However, only the pressing portions 69 may be provided. That is, an endoscope having a plurality of channels provided therein may be constructed to use the pressing forceps 67.

In the first to fourth embodiments, the positional relationship between the observation window 35 and the channel opening 41 may be set to be reverse to the vertical direction H. Further, the observation window 35 and the channel opening 41 may be set to have the same height in the vertical direction H.

In the present invention, the snare loop 6 is provided as the endoscopic treatment instrument 2. However, the shape, the material, and the hardness of the snare loop 6 can be properly changed.

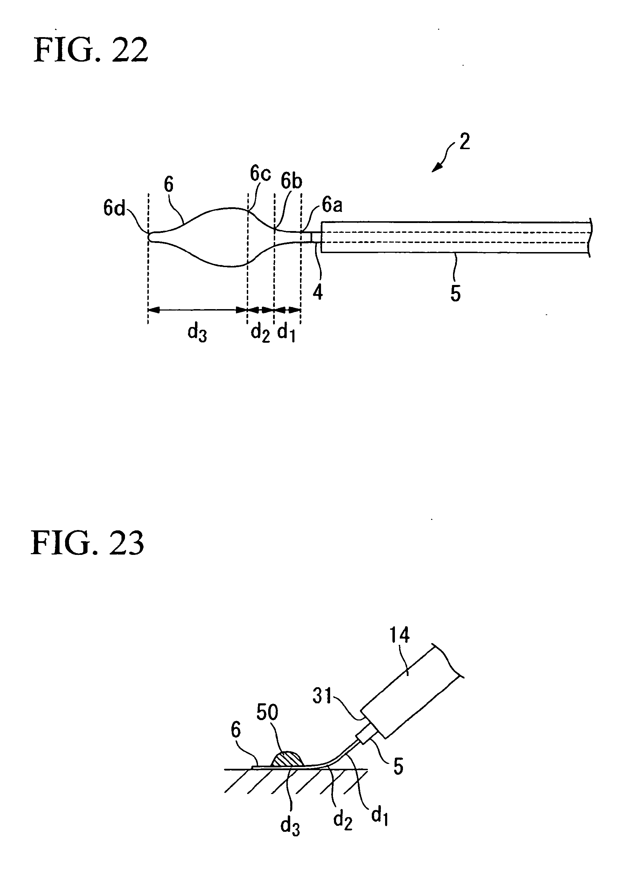

For example, as shown in FIG 22, a base end region d₁ of the snare loop 6 from the base end portion 6a to a widening start portion 6b, a widening halfway region d₂ from the widening start portion 6b to a widening halfway portion 6c, and a distal end region d₃ from the widening halfway portion 6c to a distal end portion 6d may be constructed to have different hardness. That is, the base end region d₁ and the distal end region d₃ are set to have the same hardness, but the hardness of the widening halfway region d₂ is set to be lower than that of the base end region d₁ and the distal end region d₃. That is, the widening halfway region d₂ is set to be more flexible than the other regions.

The snare loop 6 needs to have certain hardness required for excising a living tissue. However, if the hardness of the overall regions is increased, the snare loop 6 may be reflexed, when the snare loop 6 is pressed along an inner wall. Then, the distal end portion 6d floats with respect to the inner all, which makes it difficult to perform treatment.

As described above, when the widening halfway region d₂ is more flexible than the other regions, and if the snare loop 6 is pressed against the inner wall from an oblique upper side, the snare loop 6 is bent, with the distal end of the widening halfway region d₂ being set to a support point. When the snare loop 6 is further pressed, the snare loop 6 is closely attached to the inner wall, while the support point moves toward the rear end of the widening halfway region d₂. At this time, the widening halfway region d₂ can be easily bent as a whole. Therefore, as the pressing force or angle of the snare loop 6 is minutely adjusted as shown in FIG. 23, the entire snare loop 6 can be closely attached to the surface of the inner wall. Therefore, it is possible to easily perform the treatment.

Further, the observation unit is not limited to the CCD 34. For example, the observation unit may be changed into a C-MOS or image guide fiber.

In the first to fourth embodiments, the treatment has been exemplified, where the fold 51 or the lesion 61 was excised. Without being limited thereto, however, the invention can be applied to various treatments.

According to the invention, even when an obstructing tissue is present, the obstructing tissue can be easily pressed by the pressing members. Therefore, it is possible to easily secure the treatment region and to easily and rapidly perform a treatment depending on various conditions.

## Claims

1. An endoscope apparatus (1) comprising:
an endoscope insertion portion (14) including:
a treatment instrument channel (30) for inserting a treatment instrument (2);
an observation device (34) for observing a treatment target portion (45);
an observation window (35), through which observation is performed using the observation device (34) of the endoscope insertion portion (14) and formed on a distal end surface (31) of the endoscope insertion portion (14); and
a channel opening (41) of the treatment instrument channel (30) formed on the distal end surface (31) of the endoscope insertion portion (14), wherein various treatments can be performed while a captured image obtained by the observation device (34) is observed,
a pressing member (24) for pressing or supporting a portion adjacent to the treatment target portion (45), the pressing member (24) is provided to move between a projection position (B), wherein
the pressing member (24) is projected from the distal end surface (31) and a retreat position (A, C), the pressing member (24) is disposed at the distal end surface (31) of the endoscope insertion portion (14) so as to be retreated from the projection position (B), the pressing member (24) is projected from a projection opening (44) formed in the distal end surface (31) of the endoscope insertion portion (14) located closer to a periphery of the distal end surface than the channel opening (41), **characterized in that** the pressing member (24) has a pair of plate-shaped pressing rods (24) which are formed of an elastic member and configured in such a manner that the space between the pressing rods (24) is gradually widened when projecting from the distal end of the endoscope insertion portion (14), and
the lateral cross-sections of the pressing rods (24) are formed in a vertically long shape extending in the vertical direction (H).

2. The endoscope apparatus according to claim 1,
wherein the retreat position (A, C) is a withdrawal position (A) where the pressing member (24) is withdrawn into the endoscope insertion portion (14), and
the pressing member (24) is provided to reciprocate between the projection position (B) and the withdrawal position (A).

3. The endoscope apparatus according to claim 1,
wherein the channel opening (41) is provided on the distal end surface (31) so as to be closer to the projection opening (44) than the observation window (35).

4. The endoscope apparatus according to claim 1,
wherein the projection opening (44) is disposed on an opposite side of the distal end surface (31) from the observation window (35).

## Patentansprüche

1. Endoskopvorrichtung (1) aufweisend:
einen Endoskop-Einführbereich (14) mit:
einem Behandlungsinstrumentenkanal (30) zum Einführen eines Behandlungsinstruments (2);
einer Beobachtungsvorrichtung (34) zum Beobachten eines Behandlungszielbereichs (45);
einem Beobachtungsfenster (35), durch welches eine Beobachtung ausgeführt wird, indem das Beobachtungsfenster (34) des Endoskop-Einführbereichs (14) verwendet wird und das an einer distalen Endfläche (31) des Endoskop-Einführbereichs (14) ausgebildet ist; und
einer Kanalöffnung (41) des Behandlungsinstrumentenkanals (30), die an der distalen Endfläche (31) des Endoskop-Einführbereichs (14) ausgebildet ist, wobei verschiedene Behandlungen ausgeführt werden können, während ein erfasstes Bild beobachtet wird, das durch die Beobachtungsvorrichtung (34) erhalten wird,
einem Anpresselement (24) zum Pressen oder Halten eines Bereichs, der an den Behandlungszielbereich (45) angrenzt, wobei das Anpresselement (24) dazu vorgesehen ist, sich zwischen einer Vorsprungposition (B), bei der das Anpresselement (24) von der distalen Endfläche (31) vorsteht und einer Ruheposition (A, C) zu bewegen, das Anpresselement (24) an der distalen Endfläche (31) des Endoskop-Einführbereichs (14) zum Zurückziehen aus der Vorsprungposition (B) angeordnet ist, das Anpresselement (24) von einer Vorsprungöffnung (44) vorsteht, die in der distalen Endfläche (31) des Endoskop-Einführbereichs (14) ausgebildet ist und die näher an einem Umfang der distalen Endfläche als die Kanalöffnung (41) angeordnet ist, **dadurch gekennzeichnet, dass**
das Anpresselement (24) ein Paar plattenförmige Anpressstäbe (24) aufweist, die aus einem elastischen Element geformt und derart ausgebildet sind, dass der Zwischenraum zwischen den Anpressstäben (24) sich allmählich aufweitet, wenn diese vom distalen Ende des Endoskop-Einführbereichs (14) vorspringen, und
die Längsquerschnitte der Anpressstäbe (24) eine vertikal längliche Form aufweisen, die sich in vertikaler Richtung (H) erstreckt.

2. Endoskopvorrichtung nach Anspruch 1,
wobei die Ruheposition (A, C) eine Rückzugsposition (A) ist, in der das Anpresselement (24) in den Endoskop-Einführbereich (14) zurückgezogen ist,
das Anpresselement (24) dazu vorgesehen ist, sich zwischen der Vorsprungposition (B) und der Rückzugsposition (A) hin und her zu bewegen.

3. Endoskopvorrichtung nach Anspruch 1,
wobei die Kanalöffnung (41) an der distalen Endfläche (31) so vorgesehen ist, dass sie der Vorsprungöffnung (44) näher ist als das Beobachtungsfenster (35).

4. Endoskopvorrichtung nach Anspruch 1,
wobei die Vorsprungöffnung (44) an einer gegenüberliegenden Seite der distalen Endfläche (31) vom Beobachtungsfenster (35) angeordnet ist.

## Revendications

1. Appareil d'endoscope (1) comprenant:
une partie d'insertion d'endoscope (14) incluant:
un canal d'instrument de traitement (30) destiné à insérer un instrument de traitement (2);
un dispositif d'observation (34) destiné à observer une partie cible de traitement (45);
une fenêtre d'observation (35), à travers laquelle l'observation est réalisée en utilisant le dispositif d'observation (34) de la partie d'insertion d'endoscope (14) et formée sur une surface d'extrémité distale (31) de la partie d'insertion d'endoscope (14); et
une ouverture de canal (41) du canal d'instrument de traitement (30) formée sur la surface d'extrémité distale (31) de la partie d'insertion d'endoscope (14), dans laquelle divers traitements peuvent être réalisés alors qu'une image capturée obtenue par le dispositif d'observation (34) est observée,
un élément de pression (24) destiné à presser ou à supporter une partie adjacente à la partie cible de traitement (45), l'élément de pression (24) est prévu pour se déplacer entre une position de projection (B), dans lequel l'élément de pression (24) est projeté depuis la surface d'extrémité distale (31) et une position de retrait (A, C), l'élément de pression (24) est disposé au niveau de la surface d'extrémité distale (31) de la partie d'insertion d'endoscope (14) de façon à être retiré de la position de projection (B), l'élément de pression (24) est projeté depuis une ouverture de projection (44) formée dans la surface d'extrémité distale (31) de la partie d'insertion d'endoscope (14) placée plus près d'une périphérie de la surface d'extrémité distale que de l'ouverture de canal (41), **caractérisé en ce que** l'élément de pression (24) comporte une paire de tiges de pression en forme de plaques (24) qui sont formées d'un élément élastique et configurées d'une manière telle que l'espace entre les tiges de pression (24) est progressivement élargi lors de la projection depuis l'extrémité distale de la partie d'insertion d'endoscope (14), et
les sections transversales latérales des tiges de pression (24) sont formées dans une forme verticalement longue s'étendant dans la direction verticale (H).

2. Appareil d'endoscope selon la revendication 1,
dans lequel la position de retrait (A, C) est une position de retrait (A) où l'élément de pression (24) est retiré dans la partie d'insertion (14), et
l'élément de pression (24) est prévu pour effectuer des mouvements de va-et-vient entre la position de projection (B) et la position de retrait (A).

3. Appareil d'endoscope selon la revendication 1,
dans lequel l'ouverture de canal (41) est prévue sur la surface d'extrémité distale (31) de façon à être plus près de l'ouverture de projection (44) que de la fenêtre d'observation (35).

4. Appareil d'endoscope selon la revendication 1,
dans lequel l'ouverture de projection (44) est disposée sur le côté opposé de la surface d'extrémité distale (31) depuis la fenêtre d'observation (35).
